(19) Europäisches Patentamt
European Patent Office
Office européen des brevets

(11) Numéro de publication : **0 090 733**
**B1**

(12) # FASCICULE DE BREVET EUROPÉEN

(45) Date de publication du fascicule du brevet : **30.10.85**

(21) Numéro de dépôt : **83400623.1**

(22) Date de dépôt : **25.03.83**

(51) Int. Cl.[4] : **C 07 D401/04**, C 07 D405/14, C 07 D409/14, C 07 D403/04, A 61 K 31/53, A 61 K 31/495 // (C07D401/04, 211:36, 239:50, 251:54),(C07D403/04, 207:10, 205:04, 251:54),(C07D405/14, 211:36, 251:54, 307:81, 317:58, 319:18),(C07D409/14, 211:36, 251:54, 333:58)

(54) **Polyméthylènes-imines disubstituées, leurs procédés de préparation et les compositions pharmaceutiques les renfermant.**

(30) Priorité : **30.03.82 FR 8205373**

(43) Date de publication de la demande :
**05.10.83 Bulletin 83/40**

(45) Mention de la délivrance du brevet :
**30.10.85 Bulletin 85/44**

(84) Etats contractants désignés :
**AT BE CH DE FR GB IT LI LU NL SE**

(56) Documents cités :
**US-A- 3 270 014**
**US-A- 3 362 221**
**Le dossier contient des informations techniques présentées postérieurement au dépôt de la demande et ne figurant pas dans le présent fascicule.**

(73) Titulaire : **ADIR**
**22, rue Garnier**
**F-92200 Neuilly-sur-Seine (FR)**

(72) Inventeur : **Dhainaut, Alain**
**7 rue des Guipières**
**F-78400 Chatou (FR)**
Inventeur : **Laubie, Michel**
**35 avenue Foch**
**F-92420 Vaucresson (FR)**
Inventeur : **Duhault, Jacques**
**14 bis rue Paul Demange**
**F-78290 Croissy-sur-Seine (FR)**
Inventeur : **Regnier, Gilbert**
**Avenue du Plessis**
**F-92200 Chatenay Malabry (FR)**

(74) Mandataire : **Reverbori, Marcelle et al**
**ADIR 22 Rue Garnier**
**F-92200 Neuilly-sur-Seine (FR)**

**EP 0 090 733 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet des polyméthylène-imines disubstituées, leurs procédés de préparation et les compositions pharmaceutiques les renfermant.

L'état de la technique peut être illustré par les brevets US 3 382 221 décrivant des résines de structure triazinique et US 3 270 014 relatif à des dihydrotriazines antihypertensives.

L'invention concerne particulièrement les polyméthylène-imines disubstituées de formule générale I :

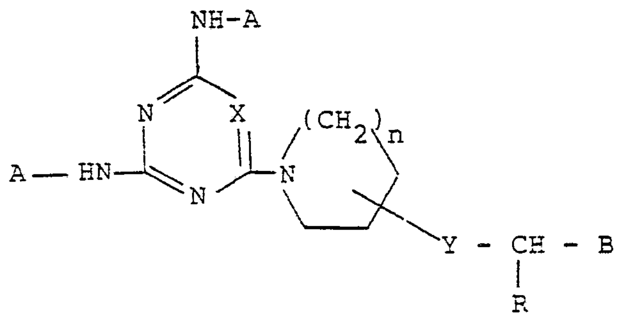

(I)

dans laquelle :

A représente un radical propyle, allyle ou butène-2 yle ;

X représente le groupe —CH— ou un atome d'azote ;

n représente zéro ou les nombres entiers 1 ou 2 ;

Y représente un atome d'oxygène ou le radical N—$R_1$ dans lequel $R_1$ représente un atome d'hydrogène, un radical alcoyle ou hydroxyalcoyle ayant chacun de 1 à 5 atomes de carbone, un radical alcényle ayant de 2 à 5 atomes de carbone, un radical cycloalcoyle ou cycloalcényle ayant chacun de 3 à 7 atomes de carbone ou un radical acétyle ;

R représente un atome d'hydrogène, un radical alcoyle ayant de 1 à 5 atomes de carbone, un radical cyclo-alcoyle ayant de 5 à 7 atomes de carbone, ou un radical phényle éventuellement substitué par un ou plusieurs atomes de fluor ou de chlore.

B représente :

un atome d'hydrogène,

un radical alcoyle ayant de 1 à 5 atomes de carbone,

un radical alcényle ayant de 2 à 5 atomes de carbone ou,

un radical phényle, naphtyle, benzofurannyle, benzothiényle, benzodioxolyle, benzodioxannyle, benzodioxinnyle, $\Delta_3$-chroményle, thio-chroményle, ou chromannyle, chacun éventuellement substitué par un ou plusieurs atomes de fluor ou de chlore ou radicaux alcoyle ou alcoxy ayant chacun de 1 à 5 atomes de carbone, ou

un radical de formule :

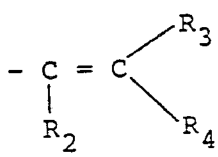

dans laquelle :

$R_2$, $R_3$ et $R_4$ identiques ou différents représentent chacun un atome d'hydrogène ou un radical phényle éventuellement substitué par un ou plusieurs atomes de chlore ou de fluor.

La présente invention a également pour objet le procédé de préparation des dérivés de formule générale I caractérisé en ce que l'on condense

une polyméthylène-imine disubstituée de formule générale II

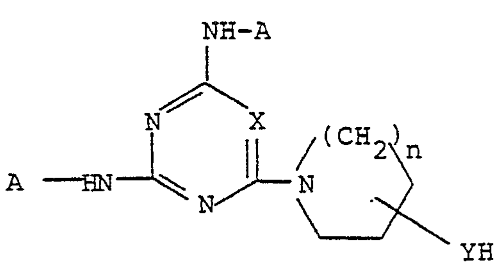

(II)

dans laquelle A, n, X, et Y ont les définitions énoncées précédemment,

avec un dérivé halogéné de formule générale III :

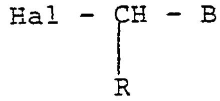

(III)

dans laquelle R et B ont la signification précédemment définie et Hal représente un atome d'halogène tel que par exemple un atome de chlore ou de brome.

La condensation s'effectue de préférence dans un solvant choisi parmi les hydrocarbures benzéniques à haut point d'ébullition comme le toluène ou le xylène, les amides aliphatiques comme le diméthylformamide ou le diméthylacétamide éventuellement mélangés à un hydrocarbure benzénique à haut point d'ébullition, ou le cyanure de méthyle. Il est avantageux d'opérer à une température comprise entre 120 et 140 °C en présence d'un accepteur de l'hydracide formé au cours de la réaction. Cet accepteur peut être choisi parmi les carbonates alcalins comme le carbonate de potassium, la triéthylamine ou un excès de la polyméthylène-imine de formule II utilisée pour la condensation.

La présente invention a aussi pour objet le procédé de préparation de dérivés de formule générale I'

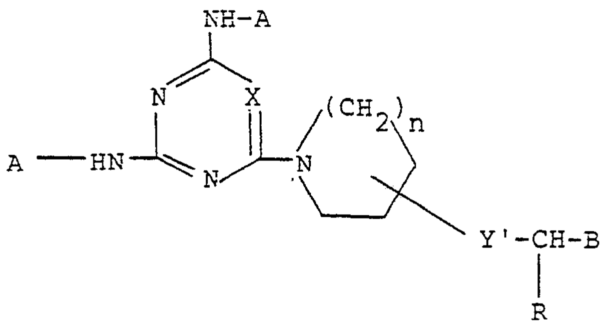

(I')

dans laquelle :

A, X, n, R et B sont tels que précédemment définis et

Y' représente un atome d'oxygène ou un radical $—N—R'_1$ dans lequel $R'_1$ représente un radical alcoyle ou hydroxyalcoyle ayant chacun de 1 à 5 atomes de carbone, un radical alcényle ayant de 2 à 5 atomes de carbone, un radical cycloalcoyle ou cycloalcényle ayant chacun de 3 à 7 atomes de carbone, ou un radical acétyle, caractérisé en ce que l'on condense :

un dérivé halogéné de formule générale IV

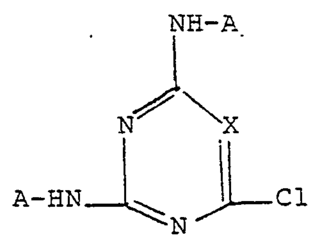

(IV)

dans laquelle A et X ont les significations précédemment définies,

avec une polyméthylène-imine monosubstituée de formule générale V

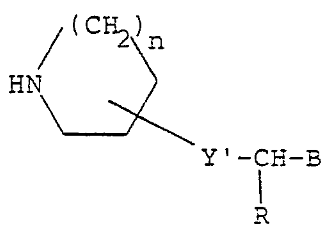

(V)

dans laquelle n, R et Y' sont tels que précédemment définis.

La condensation s'effectue de façon particulièrement adéquate dans un solvant choisi parmi les alcools en $C_4$ ou $C_5$ tels que le butanol ou le pentanol, et les amides aliphatiques comme le diméthylformamide ou le diméthylacétamide. Il est recommandé d'opérer à une température comprise entre 120 et 150 °C en présence d'un accepteur de l'hydracide formé au cours de la réaction. Cet

accepteur peut être choisi parmi un excès de l'imine de formule générale V précédemment définie, la triéthylamine ou les carbonates alcalins comme le carbonate de potassium.

La présente invention a aussi pour objet le procédé de préparation des dérivés de formule générale I''

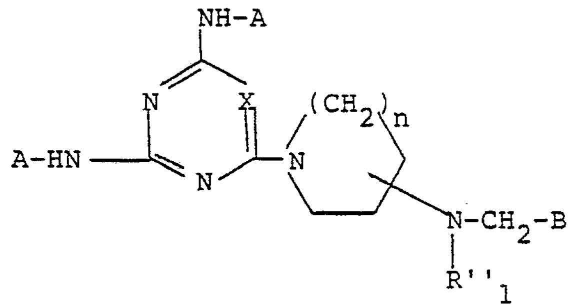

(I'')

dans laquelle :

A, X, n et B sont tels que précédemment définis et

$R''_1$ représente un atome d'hydrogène, un radical alcoyle ou hydroxyalcoyle ayant chacun de 1 à 5 atomes de carbone, un radical alcényle ayant de 2 à 5 atomes de carbone, ou un radical cycloalcoyle ou cycloalcényle ayant chacun de 3 à 7 atomes de carbone,

caractérisé en ce que l'on réduit l'amide correspondante de formule générale VI :

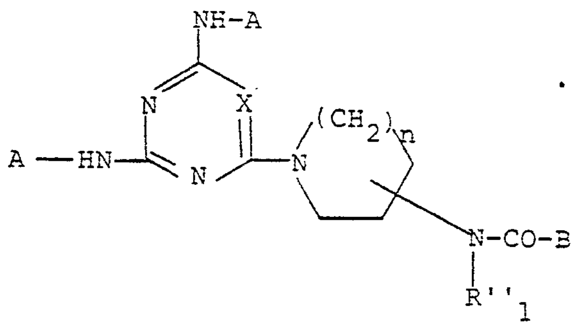

(VI)

dans laquelle A, X, n, $R''_1$ et B sont tels que définis précédemment.

Comme agent réducteur on peut utiliser par exemple l'hydrure double de lithium aluminium : $Li\,Al\,H_4$ ou l'hydrure de bore : $B_2H_6$.

Une mise en œuvre particulièrement adéquate consiste à effectuer la réduction dans un solvant comme le tétrahydrofuranne à une température comprise entre 20 et 60 °C.

La présente invention a également pour objet le procédé de préparation des dérivés de formule générale (I''')

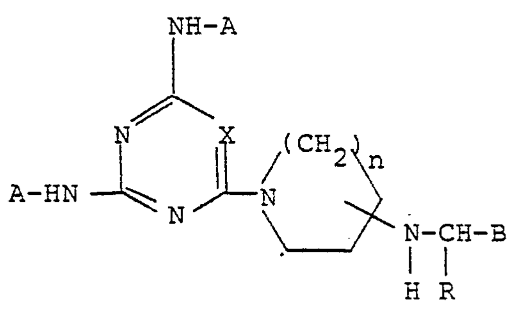

(I''')

dans laquelle :

A, X, n, R et B ont les significations précédemment définies, caractérisé en ce que l'on traite par le cyanoborohydrure de sodium un mélange de la cétone de formule VII :

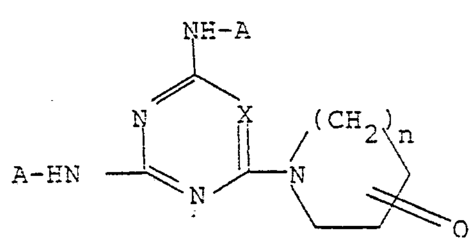

(VII)

dans laquelle A, X et n sont tels que précédemment définis et de l'amine de formule VIII :

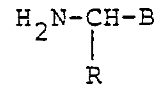

(VIII)

dans laquelle R et B sont tels que précédemment définis.

Il est particulièrement avantageux d'opérer dans un solvant approprié comme par exemple le méthanol ou le tétrahydrofuranne à une température comprise entre 20 et 25 °C à un pH voisin de 6.

Ces nouveaux dérivés ainsi obtenus peuvent être transformés en sels d'addition avec les acides, sels qui font, à ce titre, partie de l'invention. Comme acides utilisables pour la formation de ces sels, on peut citer, par exemple, dans la série minérale les acides chlorhydrique, bromhydrique, sulfurique, phosphorique et dans la série organique, les acides acétique, propionique, maléique, fumarique, tartrique, nitrique, oxalique, benzoïque, méthane sulfonique et iséthionique.

Ces nouveaux dérivés peuvent être purifiés par des méthodes physiques telles que cristallisation, chromatographie ou chimiques telles que formation de sels d'addition avec des acides et décompositions de ces sels par des agents alcalins.

Les matières premières utilisées dans les procédés précédemment décrits sont soit des produits connus, soit des produits préparés à partir de substances connues, selon des procédés décrits pour préparer des produits analogues comme indiqué dans les exemples suivants.

Les dérivés de formule générale I et leurs sels d'addition physiologiquement tolérables possèdent des propriétés pharmacologiques et thérapeutiques intéressantes. En particulier, ils favorisent la captation d'oxygène et permettent ainsi leur utilisation comme médicament, notamment dans le traitement de tout type d'hypoxie tissulaire.

Ces dérivés et leurs sels physiologiquement tolérables présentent de plus une très faible toxicité.

L'effet des dérivés de l'invention sur la pression d'oxygène ($P/O_2$) a été étudiée chez le chien anesthésié au Nembutal. Des échantillons de sang sont prélevés périodiquement 2, 5, 15, 45 et 75 minutes après l'administration des composés à tester ; ils servent à la détermination du pH de la $P/O_2$ et de la $P/CO_2$.

La $P/O_2$ est mesurée sur un appareil Radiometer $BMS_3$. La lecture de la $P/O_2$ se fait sur cet appareil préalablement étalonné avec des valeurs connues, à l'aide d'une électrode de platine, ou électrode de Clark.

Les produits ont été administrés chez le chien par la voie intraveineuse à la dose de 1 mg/kg et la détermination du pourcentage d'augmentation de la teneur en oxygène du sang artériel montre que ce pourcentage peut selon les composés atteindre jusqu'à 37 %, 75 minutes après l'administration du composé.

Les produits de la présente invention se sont révélés également actifs dans le traitement de l'hypoxie anémique induite par voie chimique par injection sous-cutanée de $NaNO_2$ selon la méthode de Gibson G. E. Neurobiol Aging *2,* 165, (1981) et Biochem. Pharmacol, *28,* 747, (1979), et dans l'hypoxie hypobare selon la technique de Legeai J.M. et coll. Experientia, *37,* 292 (1981).

La présente invention a également pour objet les compositions pharmaceutiques contenant comme principe actif un dérivé de formule générale I ou un de sels physiologiquement tolérable, mélangé ou associé à un excipient pharmaceutique approprié.

Elle concerne notamment les formes dosées renfermant de 20 à 100 mg de principe actif.

Les compositions pharmaceutiques ainsi obtenues sont présentées avantageusement sous des formes dosées diverses telles que par exemple, comprimés, dragées, gélules, suppositoires, solutions injectables ou buvables. Elles peuvent être administrées par la voie orale, rectale ou parentérale aux doses de 20 à 100 mg 1 à 2 fois par jour.

Les exemples suivants illustrent l'invention.

## Exemple 1

(bis allylamino-4,6 s.triazinyl-2)-1 (bis p.fluorobenzhydrylamino)-4 pipéridine

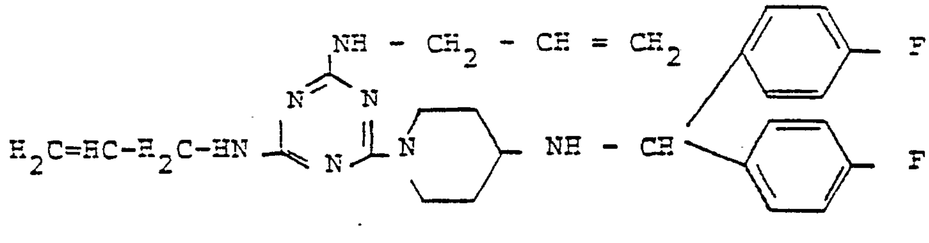

On chauffe pendant 2 heures à reflux une solution de 12,4 g de (bis allylamino-4,6 s.triazinyl-2)-1 amino-4 pipéridine, fondant (capillaire) à 118-200 °C, et de 4,04 g de bis (p.fluorophényl) bromométhane

dans 100 ml de $H_3C$ CN. Après repos une nuit à la température ordinaire, on filtre les cristaux de bromhydrate et évapore la solution sous pression réduite. On sépare le produit attendu de la base de départ en excès par filtration du résidu huileux obtenu en solution dans l'acétate d'éthyle, sur une colonne de silice. A partir du produit obtenu, après évaporation des éluats, on prépare le fumarate dans l'éthanol et obtient 5,2 g de cristaux blancs de difumarate de (bis allylamino-4,6 s.triazinyl-2)-1 (bis p.fluorobenhydrylamino)-4 pipéridine, fondant (capillaire) à 208-210 °C.

L'amino pipéridine de départ a été préparée par réduction avec Li Al $H_4$ dans le tétrahydrofuranne de l'oxime correspondante fondant (capillaire) à 180 °C, elle-même préparée à partir de la (bis allylamino-4,6 s.triazinyl-2)-1 pipéridine, P.F. (capillaire) du chlorhydrate correspondant : 219-222 °C, elle-même préparée à partir de la (bis allylamino-4,6 chloro-2) s.triazine, et de la diéthoxy-4,4 pipéridine, en présence de carbonate de potassium.

Exemples 2 à 19

Les dérivés suivants ont été préparés selon la méthode décrite dans l'exemple 1.

2) (bis allylamino-4,6 s.triazinyl-2)-1 p.fluorobenzylamino-4 pipéridine, dont le difumarate fond (Kofler) à 242 °C (éthanol anhydre).

3) (bis allylamino-4,6 s.triazinyl-2)-1 (benzofurannyl-2 méthyl amino)-4 pipéridine dont le difumarate fond (Kofler) à 207 °C (éthanol anhydre).

4) (bis allylamino-4,6 s.triazinyl-2)-1 benzhydrylamino-4 pipéridine, dont le difumarate fond (capillaire) à 227 °C (éthanol).

5) (bis allylamino-4,6 s.triazinyl-2)-1 (α-cyclohexyl p.fluorobenzyl amino)-4 pipéridine, dont le difumarate fond (Kofler) à 228 °C (éthanol anhydre).

6) (bis allylamino-4,6 s.triazinyl-2)-1 pipéronylamino-4 pipéridine dont le difumarate fond (Kofler) à 227 °C (éthanol anhydre).

7) (bis crotylamino-4,6 s.triazinyl-2)-1 (bis p.fluorobenzhydrylamino)-4 pipéridine, dont le dichlorhydrate fond (Kofler) à 228 °C-230 °C.

8) (bis allylamino-2,4 pyrimidinyl-6)-1 (bis p.fluorobenzhydrylamino)-4 pipéridine, dont le dichlorhydrate fond à 240 °C (éthanol anhydre).

9) (bis allylamino-4,6 s.triazinyl-2)-1 (N-bis p.fluorobenzhydryl N-éthyl amino)-4 pipéridine, dont le difumarate fond (Kofler) à 163 °C (éthanol anhydre).

10) (bis allylamino-4,6 s.triazinyl-2)-1 p.fluorobenzhydrylamino-4 pipéridine, dont le dichlorhydrate fond (Kofler) à 242 °C (éthanol anhydre).

11) (bis allylamino-4,6 s.triazinyl-2)-1 (chromène-3 yl-3 méthyl amino)-4 pipéridine, dont le dichlorhydrate fond (Kofler) à 220 °C (éthanol anhydre).

12) (bis allylamino-4,6 s.triazinyl-2)-1 cinnamylamino-4 pipéridine, dont le dichlorydrate fond (Kofler) à 195 °C (éthanol anhydre).

13) (bis allylamino-4,6 s.triazinyl-2)-1 (fluoro-4 naphtyl-1 méthyl amino)-4 pipéridine dont le difumarate fond (Kofler) à 217 °C (éthanol anhydre).

14) (bis allylamino-4,6 s.triazinyl-2)-1 (bis p.fluorobenzhydryloxy)-4 pipéridine, dont le chlorhydrate fond (capillaire) à 182-184 °C (éthanol).

15) (bis allylamino-4,6 s.triazinyl-2)-1 éthoxy-4 pipéridine, dont le difumarate fond (Kofler) à 188 °C (éthanol anhydre).

16) (bis allylamino-4,6 s.triazinyl-2)-1 (benzothiényl-2 méthyl amino)-4 pipéridine, dont le difumarate fond (Kofler) à 220 °C (éthanol à 95 %).

17) (bis allylamino-4,6 s.triazinyl-2)-1 (bis p.-fluorobenzhydrylamino)-3 pipéridine.

18) (bis allylamino-4,6 s.triazinyl-2)-1 (bis p.-fluorobenzhydrylamino)-3 azétidine.

19) (bis allylamino-4,6 s.triazinyl-2)-1 (bis p.-fluorophényl allylamino)-4 pipéridine.

Exemple 20

(bis allylamino-4,6 s.triazinyl-2)-1(N-bis p.fluorobenzhydryl N-éthyl amino)-4 pipéridine.

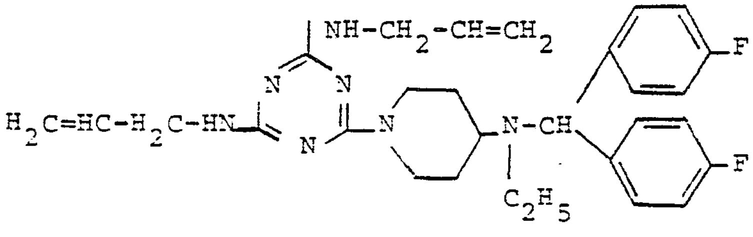

On chauffe pendant 7 heures à reflux une solution de 4,6 g de bis allylamino-4,6 chloro-2 s.triazine et de 7,8 g de N-éthyl N-(bis p.fluorobenzhydryl amino)-4 pipéridine dans 150 ml de n.butanol en présence

de 2,02 g de triéthylamine. Au bout de ce temps, on refroidit et évapore la solution sous pression réduite ; le résidu gélatineux obtenu est traité avec 250 ml de benzène et lavé avec 4 fois 50 ml d'eau. Après décantation et évaporation du benzène, on obtient 110 g d'huile résiduelle dont on prépare le fumarate au sein de l'éthanol. On obtient 11,2 g de difumarate fondant (capillaire) à 163 °C.

L'amino pipéridine de départ (Eb/≃ 10 Pa : 190-200 °C) a été préparée par désacétylation, au moyen de soude dans l'éthanol, de l'acétyl-1 (N-éthyl, N-bis p.fluorobenzhydryl amino)-4 pipéridine, (huile) elle-même préparée par condensation dans l'acétonitrile du bromure de bis p.fluorobenzhydryle et de l'acétyl-1 éthylamino-4 pipéridine (Eb/≃ $2.10^3$ Pa : 155-160 °C). Rendement : 74 %.

## Exemples 21 à 26

Les dérivés suivants ont été préparés selon la méthode décrite dans l'exemple 20 :

21) (bis allylamino-4,6 s.triazinyl-2)-1 (N-bis p.fluorobenzhydryl N-allyl amino)-4 pipéridine.

22) (bis allylamino-4,6 s.triazinyl-2)-1 (N-bis p.fluorobenzhydryl N-cyclohexyl amino)-4 pipéridine.

23) (bis allylamino-4,6 s.triazinyl-2)-1 (N-bis p.fluorobenzhydryl N-hydroxyéthyl amino)-4 pipéridine.

24) (bis allylamino-4,6 s.triazinyl-2)-1 (N-cinnamyl N-éthyl amino)-4 pipéridine, P.F. (Kofler) du difumarate correspondant : 168-170 °C (éthanol anhydre).

25) (bis n.propylamino-4,6 s.triazinyl-2)-1 (bis p.fluorobenzhydryl amino)-4 pipéridine, P.F. (capillaire) du difumarate correspondant : 223-225 °C (éthanol anhydre).

26) (bis allylamino-4,6 s.triazinyl-2)-1(fluoro-4 naphtyl-1 acétyl amino)-4pipéridine, P.F. (capillaire) du fumarate correspondant : 147-152 °C (éthanol anhydre).

## Exemple 27

(bis allylamino-4,6 s.triazinyl-2)-1 (bis p.fluorobenzhydryloxy)-4 pipéridine.

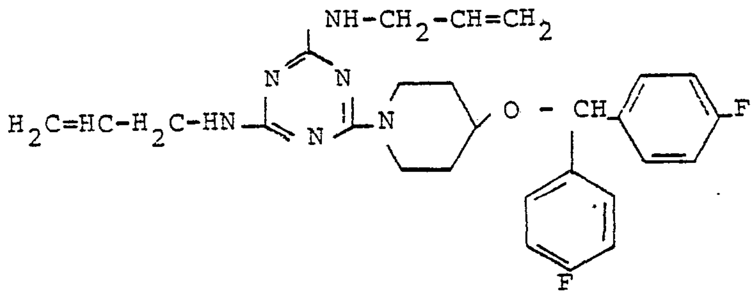

On chauffe pendant 6 heures à reflux une solution de 2,25 g de bis allylamino-4,6 chloro-2 triazine et de 3 g de bis p.fluorobenzhydryloxy-4 pipéridine dans 50 ml de butanol, en présence de 1,4 ml de triéthylamine. Au bout de ce temps, on évapore le solvant reprend le résidu par 100 ml d'éther et lave la solution avec 100 ml d'une solution à 10 % de carbonate de sodium puis 2 fois 50 ml d'eau. Après décantation et évaporation de l'éther, on obtient une huile résiduelle dont on prépare le chlorhydrate au sein de l'éthanol. On isole 2,1 g de chlorydrate de (bis allylamino-4,6 s.triazinyl-2)-1 (bis p.fluorobenzhydryloxy)-4 pipéridine fondant (capillaire) à 182-184 °C.

La bis p.fluorobenzhydryloxy-4 pipéridine, dont l'oxalate fond (capillaire) à 182-184 °C, a été préparée par condensation du bromure de bis p.fluorobenzhydryle avec l'acétyl-1 pipéridinol-4 dans le tétrahydrofuranne en présence d'hydrure de sodium.

## Exemple 28-39

Les dérivés suivants ont été préparés selon la méthode décrite dans l'exemple 27 :

28) (bis allylamino-4,6 s.triazinyl-2)-1 éthoxy-4 pipéridine, P.F. (Kofler) du difumarate correspondant : 188 °C (éthanol anhydre).

29) (bis allylamino-4,6 s.triazinyl-2)-1 méthoxy-4 pipéridine, P.F. (Kofler) du fumarate correspondant : 193 °C (éthanol).

30) (bis allylamino-4,6 s.triazinyl-2)-1 allyloxy-4 pipéridine, P.F. (Kofler) du fumarate correspondant : 184 °C (éthanol).

31) (bis allylamino-4,6 s.triazinyl-2)-1 n.butoxy-4 pipéridine, P.F. (Kofler) du fumarate correspondant : 183 °C (éthanol).

32) (bis allylamino-4,6 s.triazinyl-2)-1 n.propoxy-4 pipéridine, P.F. (Kofler) du fumarate correspondant : 195 °C (éthanol anhydre).

33) (bis allylamino-4,6 s.triazinyl-2)-1 cinnamoyloxy-4 pipéridine, P.F. (Kofler) du fumarate correspondant : 165 °C (éthanol).

34) (bis allylamino-4,6 s.triazinyl-2)-1 p.fluorocinnamyloxy-4 pipéridine, P.F. (Kofler) du fumarate correspondant : 152 °C (éthanol anhydre).

35) (bis allylamino-2,4 pyrimidinyl-6)-1 éthoxy-4 pipéridine, P.F. (Kofler) du fumarate correspondant : 158 °C (éthanol anhydre).

36) (bis allylamino-4,6 s.triazinyl-2)-1 (benzothiényl-2 méthoxy)-4 pipéridine, P.F. (Kofler) : 110 °C (acétonitrile).

37) (bis allylamino-4,6 s.triazinyl-2)-1 éthoxy-3 pipéridine, P.F. (Kofler) du fumarate correspondant : 152 °C (éthanol anhydre).

38) (bis allylamino-4,6 s.triazinyl-2)-1 éthoxy-3 pyrrolidine, P.F. (capillaire) du fumarate correspondant : 148-150 °C (éthanol anhydre).

39) (bis allylamino-4,6 s.triazinyl-2)-1 éthoxy-3 azétidine, P.F. (Kofler) : 96 °C.

Exemple 40

(bis allylamino-4,6 s.triazinyl-2)-1 (benzofurannyl-2 méthylamino)-4 pipéridine :

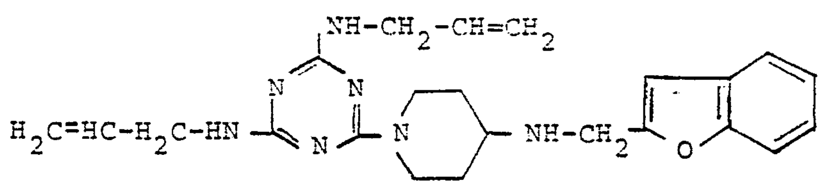

On chauffe pendant 30 heures à reflux une solution de 8 g de (bis allylamino-4,6 s.triazinyl-2)-1-(benzofurannyl-2 carbonamido)-4 pipéridine, fondant (Kofler) à 160 °C, dans 250 ml de tétrahydrofuranne en présence de 4,2 g et d'alanate de lithium. Ensuite on refroidit à 0 °C et ajoute successivement, sous azote, 4 ml d'eau, 8 ml de solution 2N de soude de 12 ml d'eau, puis on essore le précipité d'alumine formé. On rince plusieurs fois avec le tétrahydrofuranne et évapore le filtrat à sec. On obtient une huile qu'on reprend par $CH_2Cl_2$ et sèche sur $Na_2SO_4$. On évapore à nouveau le solvant et obtient 7,2 g d'une huile jaune que l'on purifie par filtration de la solution dans l'acétate d'éthyle, sur silice. Les éluats évaporés donnent 3 g de produit que l'on transforme en fumarate au sein de l'éthanol. On isole finalement 4,1 g de difumarate de (bis allylamino-4,6 s.triazinyl-2)-1 (benzofurannyl-2 méthyl amino)-4 pipéridine sous forme de cristaux blancs fondant (Kofler) à 207 °C.

L'amide de départ a été préparée par addition du chlorure d'acide benzofuranne-2 carboxylique sur la (bis allylamino-4,6 s. triazinyl-2)-1 amino-4 pipéridine, (dont le dichlorhydrate fond au-dessus de 260 °C), dans le tétrahydrofuranne en présence de triéthylamine.

Exemples 41 à 46

Les dérivés suivants ont été préparés selon la méthode décrite dans l'exemple 40 :

41) (bis allylamino-4,6 s.triazinyl-2)-1 p.fluorobenzylamino-4 pipéridine, P.F. (Kofler) du difumarate correspondant : 242 °C (éthanol anhydre).

42) (bis allylamino-4,6 s.triazinyl-2)-1 pipéronylamino-4 pipéridine, P.F. (Kofler) du difumarate correspondant : 227 °C (éthanol anhydre).

43) (bis allylamino-4,6 s.triazinyl-2)-1 (chromène-3 yl-3 méthyl amino)-4 pipéridine, P.F. (Kofler) du dichlorhydrate correspondant : 220 °C (éthanol anhydre).

44) (bis allylamino-4,6 s.triazinyl-2)-1 cinnamylamino-4 pipéridine P.F. (Kofler) du dichlorhydrate correspondant : 195 °C (éthanol anhydre).

45) (bis allylamino-4,6 s.triazinyl-2)-1 (fluoro-4 naphtyl-1 méthyl amino)-4 pipéridine, P.F. (Kofler) du difumarate correspondant : 217 °C (éthanol anhydre).

46) (bis allylamino-4,6 s.triazinyl-2)-1 (benzothiényl-2 méthyl amino)-4 pipéridine, P.F. (Kofler) du difumarate correspondant : 220 °C (éthanol à 95 %).

Exemple 47

(bis allylamino-4,6 s.triazinyl-2)-1 (bis p.fluorobenzhydrylamino)-4 pipéridine :

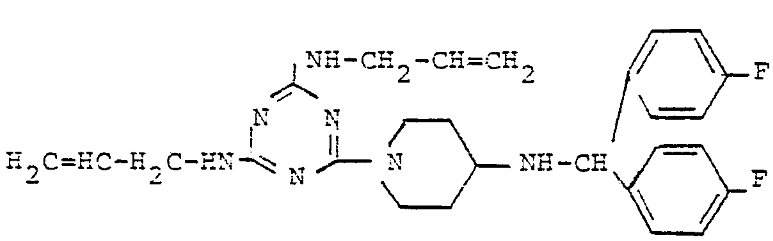

A une solution de 6,5 g de chlorhydrate de bis allylamino-4,6 s.triazinyl-2)-1 pipéridone-4 fondant (capillaire) à 219-222 °C et de 6,6 g de bis p.fluorobenzhydrylamine (Eb/0,005 mm Hg : 140-145 °C ; $n_D^{21}$ : 1,555) dans 80 ml de méthanol anhydre, on ajoute 2 g de tamis moléculaire (3 Å) et 1,25 g de cyanoborohydrure de sodium. Le pH de la suspension obtenue est ajusté à 6 avec une solution méthanolique 4N d'HCl et le mélange est agité pendant 18 heures à température ambiante. Au bout de ce temps, le solvant est évaporé et le résidu est traité avec 200 ml de chloroforme et 200 ml de bicarbonate de soude à 10 %. Après décantation, le chloroforme est évaporé et le résidu huileux pesant 9 g est transformé en difumarate au sein de l'éthanol. On obtient 9,8 g de difumarate de (bis allylamino-4,6 s.triazinyl-2)-1 (bis p.fluorobenzhydrylamino)-4 pipéridine sous forme de cristaux beiges fondant (capillaire) à 208-210 °C.

La bis p.fluorobenzhydrylamine de départ a été préparée par réduction de l'oxime de la bis p.fluorobenzophénone, fondant à 128 °C, avec de l'hydrogène, en présence de nickel de Raney, sous une pression de 50 atmosphères.

Exemples 48 à 68

Les dérivés suivants ont été préparés selon le procédé décrit dans l'exemple 47 :

48) (bis allylamino-4,6 s.triazinyl-2)-1 p.fluorobenzylamino-4 pipéridine, P.F. (Kofler) du difumarate correspondant : 242 °C (éthanol anhydre).

49) (bis allylamino-4,6 s.triazinyl-2)-1 (benzofurannyl-2 méthyl amino)-4 pipéridine, P.F. (Kofler) du difumarate correspondant : 207 °C (éthanol anhydre).

50) (bis allylamino-4 s.triazinyl-2)-1 benzhydrylamino-4 pipéridine, P.F. (capillaire) du difumarate correspondant : 227 °C (éthanol).

51) (bis allylamino-4,6 s.triazinyl-2)-1 (α-cyclohexyl p.fluorobenzyl amino)-4 pipéridine, P.F. (Kofler) du difumarate correspondant : 228 °C (éthanol anhydre).

52) (bis allylamino-4,6 s.triazinyl-2)-1 pipéronylamino-4 pipéridine, P.F. (Kofler) du difumarate correspondant : 227 °C (éthanol anhydre).

53) (bis crotylamino-4,6 s.triazinyl-2)-1 (bis p.fluorobenzhydrylamino)-4 pipéridine, P.F. (Kofler) du dichlorhydrate correspondant : 228-230 °C.

54) (bis allylamino-2,4 pyrimidinyl-6)-1 (bis p.fluorobenzhydryl amino)-4 pipéridine, P.F. (Kofler) du dichlorhydrate correspondant : 240 °C (éthanol anhydre).

55) (bis allylamino-4,6 s.triazinyl-2)-1 p.fluorobenzhydrylamino-4 pipéridine, P.F. (Kofler) du dichlorhydrate correspondant : 242 °C (éthanol anhydre).

56) (bis allylamino-4,6 s.triazinyl-2)-1 (chromène-3 yl-3 méthylamino)-4 pipéridine, P.F. (Kofler) du dichlorhydrate correspondant : 220 °C (éthanol anhydre).

57) (bis allylamino-4,6 s.triazinyl-2)-1 cinnamylamino-4 pipéridine, P.F. (Kofler) du dichlorhydrate correspondant : 195 °C (éthanol anhydre).

58) (bis allylamino-4,6 s.triazinyl-2)-1 (fluoro-4 naphtyl-1 méthylamino)-4 pipéridine, P.F. (Kofler) du difumarate correspondant : 217 °C (éthanol anhydre).

59) (bis allylamino-4,6 s.triazinyl-2)-1 (benzothiényl-2 méthyl amino)-4 pipéridine, P.F. (Kofler) du difumarate correspondant : 220 °C (éthanol à 95 %).

60) (bis allylamino-2,4 pyrimidinyl-6)-1 cinnamylamino-4 pipéridine, P.F. (Kofler) : 205 °C (éthanol).

61) (bis allylamino-4,6 s.triazinyl-2)-1 bis p.fluorobenzhydrylamino-3 pipéridine, P.F. (Kofler) du difumarate correspondant : 150 °C (éthanol anhydre).

62) (bis allylamino-4,6 s.triazinyl-2)-1 (benzodioxinyl-6 méthyl amino)-4 pipéridine, P.F. (Kofler) du difumarate correspondant : 220 °C (éthanol anhydre).

63) (bis allylamino-4,6 s.triazinyl-2)-1 p.fluorocinnamylamino-4 pipéridine, P.F. (Kofler) du difumarate correspondant : 210 °C (méthanol anhydre).

64) (bis allylamino-4,6 s.triazinyl-2)-1 éthylamino-4 pipéridine, P.F. (Kofler) : 125 °C (acétonitrile).

65) (bis allylamino-2,4 pyrimidinyl-6)-1 p. fluorocinnamylamino-4 pipéridine P.F. (Kofler) du difumarate correspondant : 195 °C (éthanol anhydre).

66) (bis allylamino-4,6 s.triazinyl-2)-1 (bis p.fluorophényl-3,3 allyl amino)-4 pipéridine, P.F. (Kofler) du difumarate correspondant : 228 °C (éthanol anhydre).

67) (bis allylamino-4,6 s.triazinyl-2)-1 cinnamylamino-3 pipéridine, P.F. (Kofler) du fumarate correspondant : 220 °C (éthanol).

68) (bis allylamino-4,6 s.triazinyl-2)-1 cinnamylamino-3 pyrrolidine, P.F. (capillaire) du difumarate correspondant : 206-209 °C (éthanol anhydre).

**Revendications** (pour les Etats contractants : BE, CH, DE, FR, GB, IT, LI, LU, NL, SE)

1. Les polyméthylènes-imines disubstituées de formule générale I :

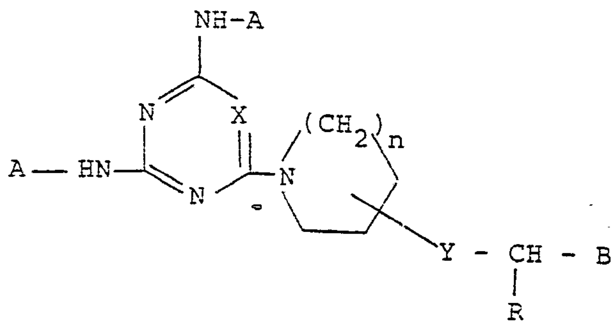

(I)

dans laquelle :

A représente un radical propyle, allyle ou butène-2yle ;

X représente le groupe —CH— ou un atome d'azote ;

n représente zéro ou les nombres entiers 1 ou 2 ;

Y représente un atome d'oxygène ou le radical N-$R_1$ dans lequel $R_1$ représente un atome d'hydrogène, un radical alcoyle ou hydroxyalcoyle ayant chacun de 1 à 5 atomes de carbone, un radical alcényle ayant de 2 à 5 atomes de carbone, un radical cycloalcoyle ou cycloalcényle ayant chacun de 3 à 7 atomes de carbone, ou un radical acétyle ;

R représente un atome d'hydrogène, un radical alcoyle ayant de 1 à 5 atomes de carbone, un radical cycloalcoyle ayant de 5 à 7 atomes de carbone ou un radical phényle éventuellement substitué par un ou plusieurs atomes de fluor ou de chlore ; et

B représente :

un atome d'hydrogène ;

un radical alcoyle ayant de 1 à 5 atomes de carbone ;

un radical alcényle ayant de 2 à 5 atomes de carbone ;

un radical phényle, naphtyle, benzofurannyle, benzothiényle, benzodioxolyle, benzodioxannyle, benzodioxinnyle, $\Delta_3$-chroményle, thio-chroményle ou chromannyle, chacun éventuellement substitué par un ou plusieurs atomes de fluor ou de chlore ou radicaux alcoyle ou alcoxy ayant chacun de 1 à 5 atomes de carbone, ou

un radical de formule

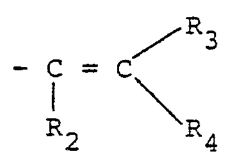

dans laquelle :

$R_2$, $R_3$ et $R_4$ identiques ou différents représentent chacun un atome d'hydrogène ou un radical phényle éventuellement substitué par un ou plusieurs atomes de chlore ou de fluor.

2. Les sels des composés de la revendication 1 avec des acides appropriés.

3. Les sels selon la revendication 2 qui sont physiologiquement tolérables.

4. La (bis allylamino-4,6 s.triazinyl-2)-1 (bis p.-fluorobenzhydrylamino)-4 pipéridine, et son difumarate.

5. La (bis allylamino-4,6 s.triazinyl-2)-1 p.fluorobenzhydrylamino-4 pipéridine et son dichlorhydrate.

6. La (bis allylamino-4,6 s.triazinyl-2)-1 éthoxy-4 pipéridine, et son difumarate.

7. La (bis allylamino-4,6 s.triazinyl-2)-1 (benzofurannyl-2 méthylamino)-4 pipéridine.

8. La (bis allylamino-4,6 s.triazinyl-2)-1 p.fluorocinnamylamino-4 pipéridine et son difumarate.

9. La (bis allylamino-2,4 pyrimidinyl-6)-1 p.fluorocinnamylamino-4 pipéridine et son difumarate.

10. Un procédé de préparation des composés de la revendication 1 caractérisé en ce que l'on condense :

une polyméthylène-imine disubstituée de formule générale II :

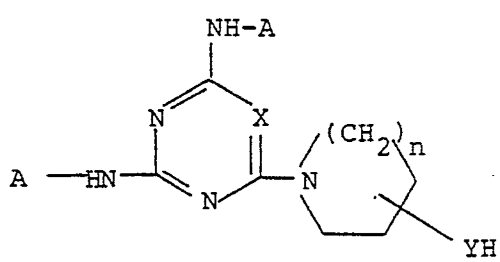

(II)

dans laquelle A, n, X, et Y sont tels que définis dans la revendication 1,

avec un dérivé halogéné de formule générale III :

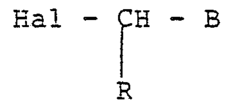

(III)

dans laquelle R et B ont les significations définies dans la revendication 1 et Hal représente un atome de chlore ou de brome.

11. Un procédé de préparation des composés de la revendication 1 répondant à la formule générale I' :

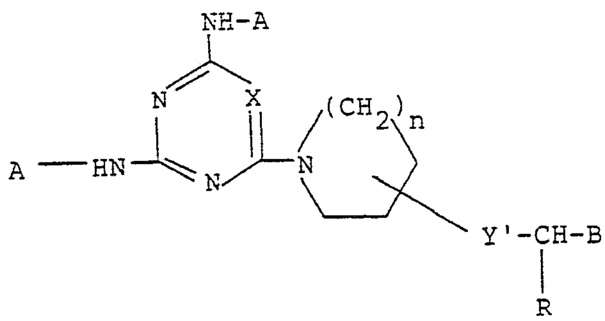

(I')

dans laquelle :

A, n, R et B ont les significations définies dans la revendication 1, et

Y' représente un atome d'oxygène ou un radical N-R'$_1$ dans lequel R'$_1$ représente un radical alcoyle ou hydroxyalcoyle ayant chacun de 1 à 5 atomes de carbone, un radical alcényle ayant de 2 à 5 atomes de carbone, un radical cycloalcoyle ou cycloalcényle ayant chacun de 3 à 7 atomes de carbone, ou un radical acétyle,

caractérisé en ce que l'on condense :

un dérivé halogéné de formule générale IV :

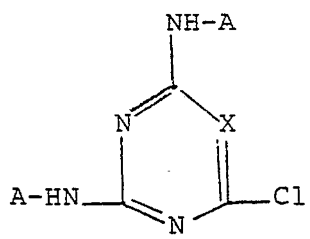

(IV)

dans laquelle A et X ont les significations précédemment définies,

avec une polyméthylène-imine monosubstituée de formule générale V

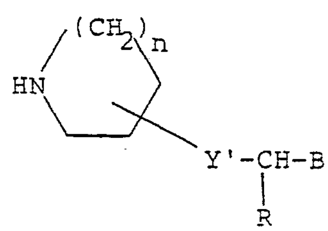

(V)

dans laquelle n, R et Y' sont tels que précédemment définis.

12. Un procédé de préparation de composés de la revendication 1 répondant à la formule générale I'' :

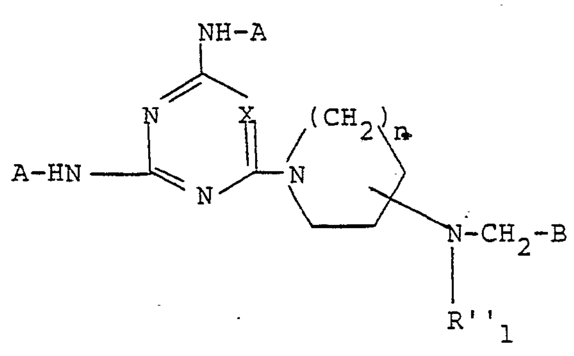

(I'')

dans laquelle A, X, n, et B ont les significations définies dans la revendication 1, et

R"$_1$ représente un atome d'hydrogène, un radical alcoyle ou hydroxyalcoyle ayant chacun de 1 à 5 atomes de carbone, un radical alcényle ayant de 2 à 5 atomes de carbone ou un radical cycloalcoyle ou cycloalcényle ayant chacun de 3 à 7 atomes de carbone ;

caractérisé en ce que l'on réduit l'amide correspondante de formule générale VI :

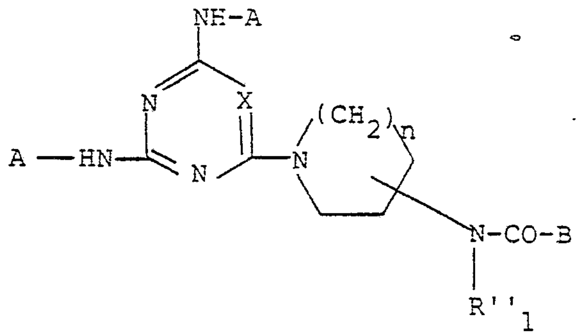

(VI)

dans laquelle A, X, n, R"$_1$ et B ont les significations précédemment définies, au moyen d'un hydrure approprié.

13. Un procédé de préparation des composés de la revendication 1 répondant à la formule générale I''' :

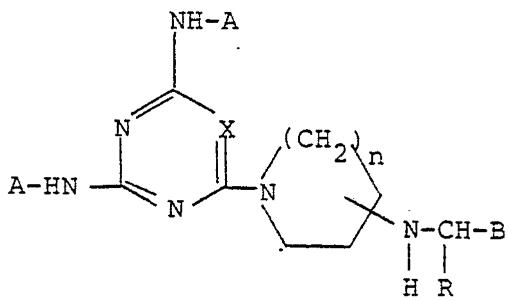

(I''')

dans laquelle A, X, n, R et B ont les significations définies dans la revendication 1,
caractérisé en ce que l'on traite par le cyanoborohydrure de sodium un mélange de cétone de formule générale VII

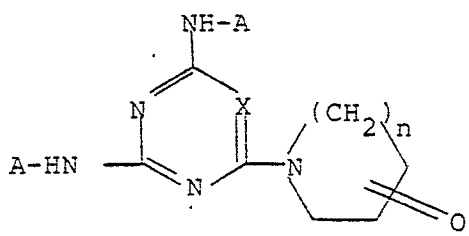

(VII)

dans laquelle A, X et n sont tels que définis dans la revendication 1, et

de l'amine de formule générale VIII

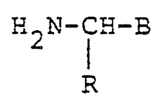

(VIII)

dans laquelle R et B ont les significations définies dans la revendication 1.

14. Les compositions pharmaceutiques contenant comme principe actif un composé selon les revendications 1 ou 3 à 9 avec les excipients pharmaceutiques appropriés.

15. Les compositions pharmaceutiques selon la revendication 14 présentées sous une forme convenant notamment pour le traitement de tout type d'hypoxie tissulaire.

**Revendications** (pour l'Etat contractant AT)

1. Procédé de préparation des polyméthylène-imines disubstituées de formule générale I :

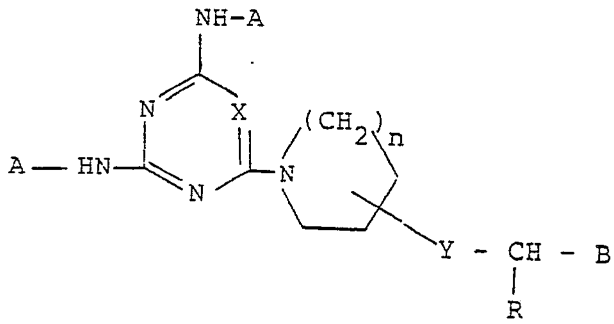

(I)

dans laquelle :

A représente un radical propyle allyle ou butène-2yle ;

X représente le groupe —CH— ou un atome d'azote ;

n représente zéro ou les nombres entiers 1 ou 2 ;

Y représente un atome d'oxygène ou le radical N-$R_1$ dans lequel $R_1$ représente un atome d'hydrogène, un radical alcoyle ou hydroxyalcoyle ayant chacun de 1 à 5 atomes de carbone, un radical alcényle ayant de 2 à 5 atomes de carbone, un radical cycloalcoyle ou cycloalcényle ayant chacun de 3 à 7 atomes de carbone, ou un radical acétyle ;

R représente un atome d'hydrogène, un radical alcoyle ayant de 1 à 5 atomes de carbone, un radical cycloalcoyle ayant de 5 à 7 atomes de carbone ou un radical phényle éventuellement substitué par un ou plusieurs atomes de fluor ou de chlore ; et

B représente :

un atome d'hydrogène ;

un radical alcoyle ayant de 1 à 5 atomes de carbone ;

un radical alcényle ayant de 2 à 5 atomes de carbone ;

un radical phényle, naphtyle, benzofurannyle, benzothiényle, benzodioxolyle, benzodioxannyle, benzodioxinnyle, $\Delta_3$-chroményle, thio-chroményle ou chromannyle, chacun éventuellement substitué par un ou plusieurs atomes de fluor ou de chlore ou radicaux alcoyle ou alcoxy ayant chacun de 1 à 5 atomes de carbone, ou

un radical de formule

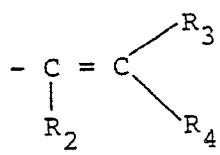

dans laquelle :

$R_2$, $R_3$ et $R_4$ identiques ou différents représentent chacun un atome d'hydrogène ou un radical phényle éventuellement substitué par un ou plusieurs atomes de chlore ou de fluor, et de leurs sels d'addition avec des acides appropriés,

caractérisé en ce que l'on condense :

une polyméthylène-imine disubstituée de formule générale II :

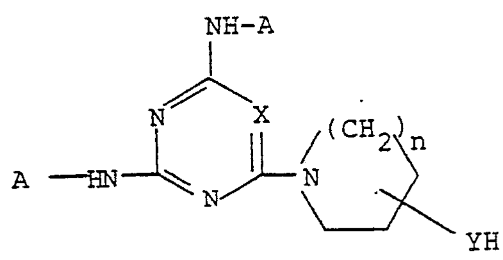

(II)

dans laquelle A, n, X et Y sont tels que définis précédemment,

avec un dérivé halogéné de formule générale III :

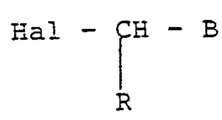

(III)

dans laquelle R et B ont les significations définies précédemment, et Hal représente un atome de chlore ou de brome ; et si on le désire on traite les dérivés I ainsi obtenus avec des acides appropriés pour donner les sels d'addition correspondants.

2. Un procédé de préparation des polyméthylène-imines disubstituées répondant à la formule générale I' :

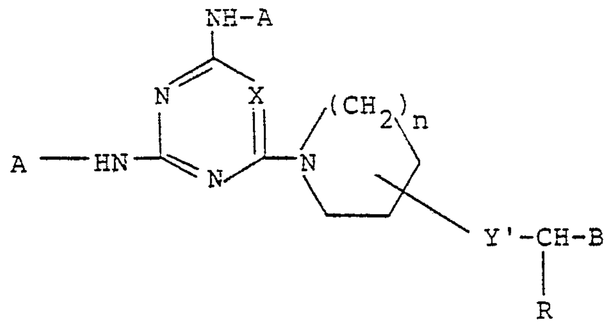

(I')

dans laquelle :

A, n, R et B ont les significations définies dans la revendication 1, et

Y' représente un atome d'oxygène ou un radical N-$R'_1$ dans lequel $R'_1$ représente un radical alcoyle ou hydroxyalcoyle ayant chacun de 1 à 5 atomes de carbone, un radical alcényle ayant de 2 à 5 atomes de carbone, un radical cycloalcoyle ou cycloalcényle ayant chacun de 3 à 7 atomes de carbone, ou un radical acétyle,

caractérisé en ce que l'on condense :

un dérivé halogéné de formule générale IV :

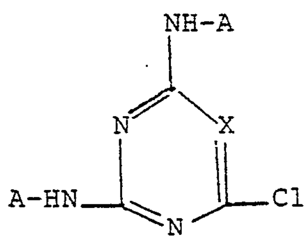

(IV)

dans laquelle A et X ont les significations définies dans la revendication 1,

avec une polymléthylène-imine monosubstituée de formule générale V

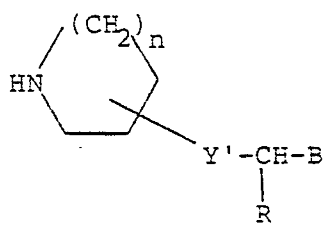

(V)

dans laquelle n, R et Y' sont tels que précédemment définis.

3. Un procédé de préparation des polyméthylène-imines disubstituées répondant à la formule générale I" :

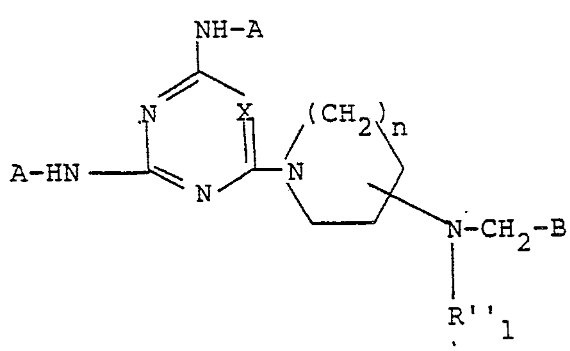

(I")

dans laquelle A, X, n, et B ont les significations définies dans la revendication 1, et

$R''_1$ représente un atome d'hydrogène, un radical alcoyle ou hydroxyalcoyle ayant chacun de 1 à 5 atomes de carbone, un radical alcényle ayant de 2 à 5 atomes de carbone ou un radical cycloalcoyle ou cycloalcényle ayant chacun de 3 à 7 atomes de carbone ;

caractérisé en ce que l'on réduit l'amide correspondante de formule générale VI :

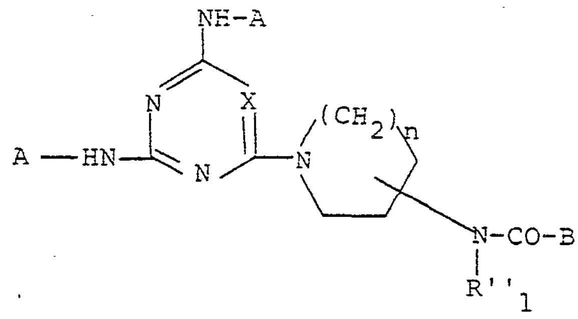

(VI)

dans laquelle A, X, n, R"$_1$ et B ont les significations précédemment définies, au moyen d'un hydrure approprié.

4. Un procédé de préparation des polyméthylène-imines disubstituées répondant à la formule générale I''' :

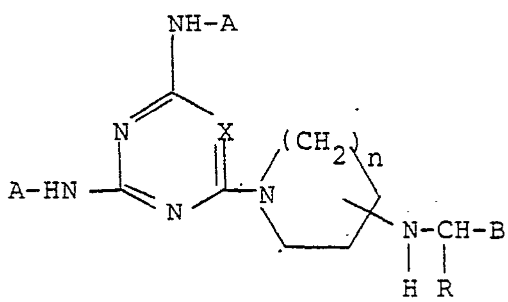

(I''')

dans laquelle A, X, n, R et B ont les significations définies dans la revendication 1, caractérisé en ce que l'on traite par le cyanoborohydrure de sodium un mélange de cétone de formule générale VII

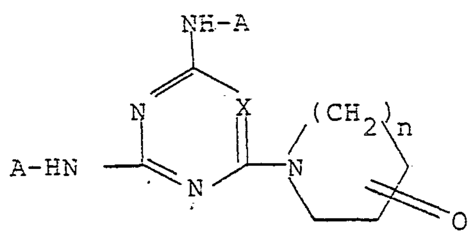

(VII)

dans laquelle A, X et n sont tels que définis dans la revendication 1, et de l'amine de formule générale VIII

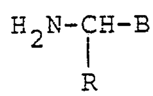

(VIII)

dans laquelle R et B ont les significations définies dans la revendication 1.

**Claims** (for the Contracting States : BE, CH, DE, FR, GB, IT, LI, LU, NL, SE)

1. Di-substituted polymethyleneimines of the general formula I :

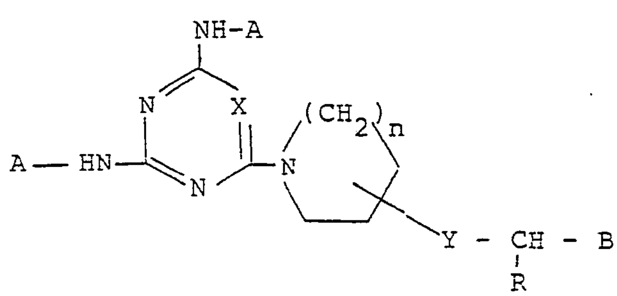

(I)

in which :

A represents a propyl, allyl or buten-2-yl radical ;

X represents the group —CH— or a nitrogen atom ;

n represents zero or the integer 1 or 2 ;

Y represents an oxygen atom or the radical $N\text{-}R_1$ in which $R_1$ represents a hydrogen atom, an alkyl or hydroxyalkyl radical each having from 1 to 5 carbon atoms, an alkenyl radical having from 2 to 5 carbon atoms, a cycloalkyl or cycloalkenyl radical each having from 3 to 7 carbon atoms, or an acetyl radical ;

R represents a hydrogen atom, an alkyl radical having from 1 to 5 carbon atoms, a cycloalkyl radical having from 5 to 7 carbon atoms or a phenyl radical optionally substituted by one or more fluorine or chlorine atoms ; and

B represents :

a hydrogen atom ;

an alkyl radical having from 1 to 5 carbon atoms ;

an alkenyl radical having from 2 to 5 carbon atoms ;

a phenyl, naphthyl, benzofuranyl, benzothienyl, benzodioxolyl, benzodioxanyl, benzodioxinyl, $\Delta_3$-chromenyl, thiochromenyl or chromanyl radical, each optionally substituted by one or more fluorine or chlorine atoms or alkyl or alkoxy radicals each having from 1 to 5 carbon atoms, or

a radical of the formula

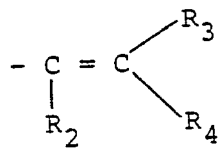

in which :

$R_2$, $R_3$ and $R_4$, which are identical or different, each represents a hydrogen atom or a phenyl radical optionally substituted by one or more chlorine or fluorine atoms.

2. The salts of the compounds of claim 1 with suitable acids.

3. The salts according to claim 2 that are physiologically tolerable.

4. 1-(4,6-bis-allylamino-2-s.-triazinyl)-4-(bis-p-fluorobenzhydrylamino)-piperidine and its difumarate.

5. 1-(4,6-bis-allylamino-2-s.-triazinyl)-4-(p-fluorobenzhydrylamino)-piperidine and its dihydrochloride.

6. 1-(4,6-bis-allylamino-2-s.-triazinyl)-4-ethoxypiperidine and its difumarate.

7. 1-(4,6-bis-allylamino-2-s.-triazinyl)-4-(2-benzofuranylmethylamino)-piperidine.

8. 1-(4,6-bis-allylamino-2-s.-triazinyl)-4-(p-fluorocinnamylamino)-piperidine and its difumarate.

9. 1-(2,4-bis-allylamino-6-pyrimidinyl)-4-(p-fluorocinnamylamino)-piperidine and its difumarate.

10. A process for the preparation of the compounds of claim 1, characterised in that :

a di-substituted polymethyleneimine of the general formula II :

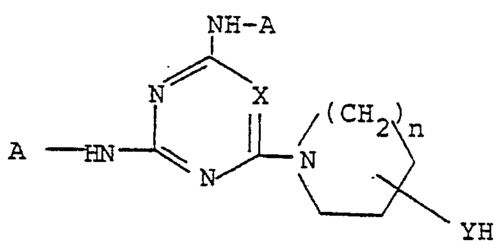

(II)

in which A, n, X and Y are as defined in claim 1, is condensed with a halogenated derivative of the general formula III :

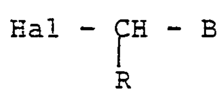

(III)

in which R and B have the meanings defined in claim 1 and Hal represents a chlorine or bromine atom.

11. A process for the preparation of the compounds of claim 1 corresponding to the general formula I' :

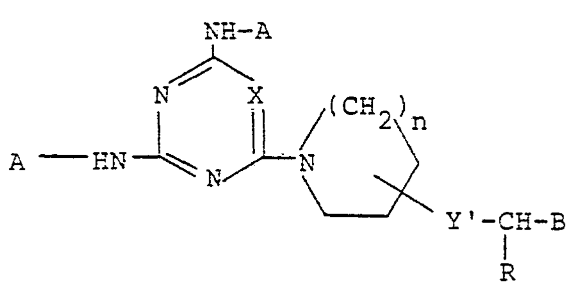

(I')

in which :

A, n, R and B have the meanings defined in claim 1 and

Y′ represents an oxygen atom or a radical N-$R'_1$ in which $R'_1$ represents an alkyl or hydroxyalkyl radical each having from 1 to 5 carbon atoms, an alkenyl radical having from 2 to 5 carbon atoms, a cycloalkyl or cycloalkenyl radical each having from 3 to 7 carbon atoms, or an acetyl radical, characterised in that :

a halogenated derivative of the general formula IV :

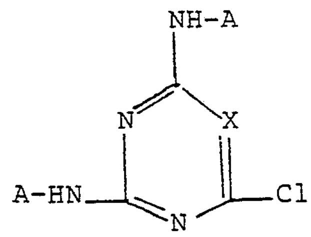

(IV)

in which A and X have the meanings defined above, is condensed

with a mono-substituted polymethyleneimine of the general formula V :

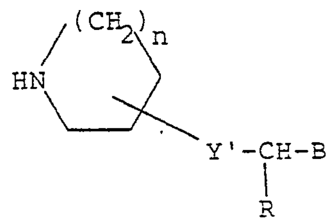

(V)

in which n, R and Y′ are as defined above.

12. A process for the preparation of the compounds of claim 1 corresponding to the general formula I″ :

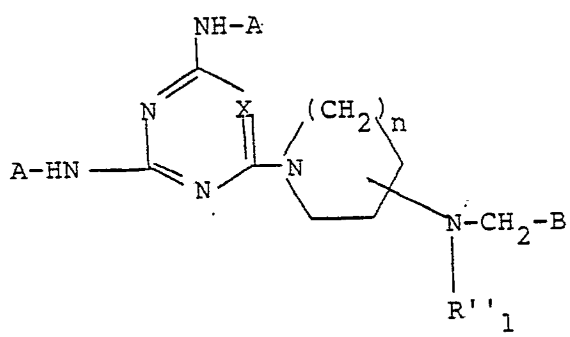

(I″)

in which A, X, n and B have the meanings defined in claim 1, and

$R''_1$ represents a hydrogen atom, an alkyl or hydroxyalkyl radical each having from 1 to 5 carbon atoms, an alkenyl radical having from 2 to 5 carbon atoms or a cycloalkyl or cycloalkenyl radical each having from 3 to 7 carbon atoms ;

characterised in that the corresponding amide of the general formula VI :

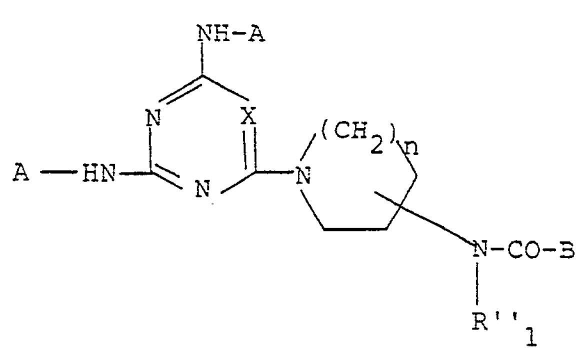

(VI)

in which A, X, n, R"$_1$ and B have the meanings defined above, is reduced by means of a suitable hydride.

13. A process for the preparation of the compounds of claim 1 corresponding to the general formula I''' :

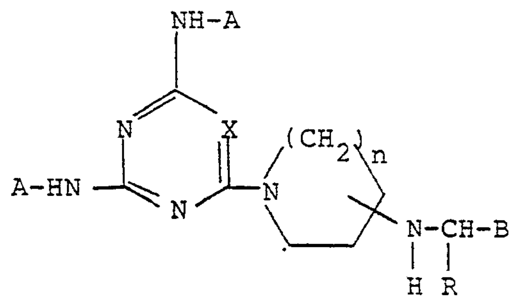

(I''')

in which A, X, n, R and B have the meanings defined in claim 1, characterised in that a mixture of a ketone of the general formula VII :

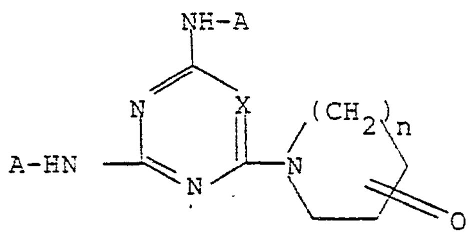

(VII)

in which A, X and n are as defined in claim 1, and an amine of the general formula VIII :

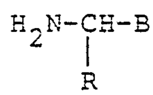

(VIII)

in which R and B have the meanings defined in claim 1, is treated with sodium cyanoborohydride.

14. Pharmaceutical compositions containing as active ingredient a compound according to claims 1 or 3 to 9 with suitable pharmaceutical excipients.

15. Pharmaceutical compositions according to claim 14 presented in a form suitable particularly for the treatment of all types of tissue hypoxia.

**Claims** (for the Contracting State AT)

1. Process for the preparation of di-substituted polymethyleneimines of the general formula I :

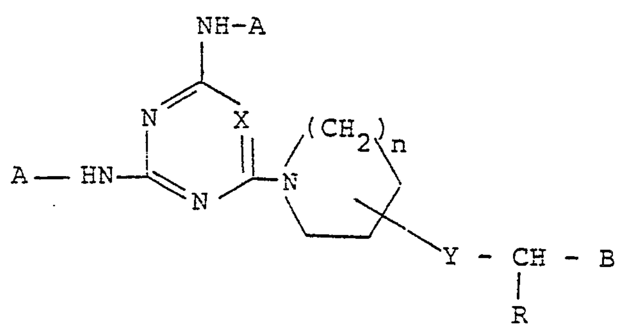

(I)

in which :

A represents a propyl, allyl or buten-2-yl radical ;

X represents the group —CH— or a nitrogen atom ;

n represents zero or the integer 1 or 2 ;

Y represents an oxygen atom or the radical N-R$_1$ in which R$_1$ represents a hydrogen atom, an alkyl or hydroxyalkyl radical each having from 1 to 5 carbon atoms, an alkenyl radical having from 2 to 5 carbon atoms, a cycloalkyl or cycloalkenyl radical each having from 3 to 7 carbon atoms, or an acetyl radical ;

R represents a hydrogen atom, an alkyl radical having from 1 to 5 carbon atoms, a cycloalkyl radical having from 5 to 7 carbon atoms or a phenyl radical optionally substituted by one or more fluorine or chlorine atoms ; and

B represents :

a hydrogen atom ;

an alkyl radical having from 1 to 5 carbon atoms ;

an alkenyl radical having from 2 to 5 carbon atoms ;

a phenyl, naphthyl, benzofuranyl, benzothienyl, benzodioxolyl, benzodioxanyl, benzodioxinyl, $\Delta_3$-chromenyl, thiochromenyl or chromanyl radical, each optionally substituted by one or more fluorine or chlorine atoms or alkyl or alkoxy radicals each having from 1 to 5 carbon atoms, or

a radical of the formula

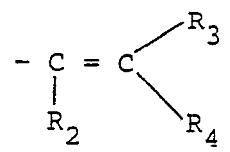

in which :

$R_2$, $R_3$ and $R_4$, which are identical or different, each represents a hydrogen atom or a phenyl radical optionally substituted by one or more chlorine or fluorine atoms, and their addition salts with suitable acids,

characterized in that :

a di-substituted polymethyleneimine of the general formula II :

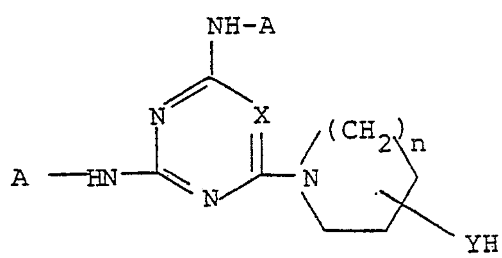

(II)

in which A, n, X and Y are as defined above, is condensed

with a halogenated derivative of the general formula III :

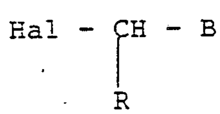

(III)

in which R and B have the meanings defined above and Hal represents a chlorine or bromine atom, and, if desired, the derivatives I so obtained are treated with suitable acids to yield the corresponding addition salts.

2. A process for the preparation of di-substituted polymethyleneimines corresponding to the general formula I' :

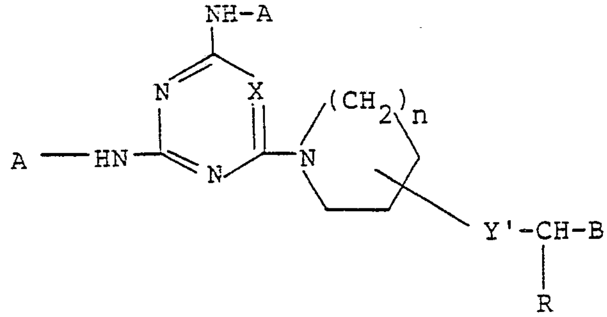

(I')

in which :

A, n, R and B have the meanings defined in claim 1 and

Y' represents an oxygen atom or a radical N-$R_1$' in which $R'_1$ represents an alkyl or hydroxyalkyl

radical each having from 1 to 5 carbon atoms, an alkenyl radical having from 2 to 5 carbon atoms, a cycloalkyl or cycloalkenyl radical each having from 3 to 7 carbon atoms, or an acetyl radical, characterized in that:

a halogenated derivative of the general formula IV:

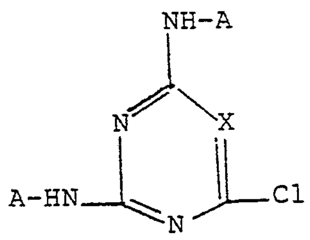

(IV)

in which A and X have the meanings defined in claim 1, is condensed with a mono-substituted polymethyleneimine of the general formula V:

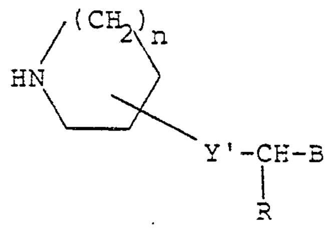

(V)

in which n, R and Y' are as defined above.

3. A process for the preparation of di-substituted polymethyleneimines corresponding to the general formula I'':

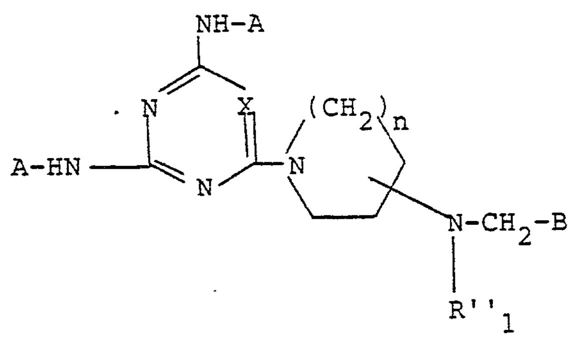

(I'')

in which A, X, n and B have the meanings defined in claim 1, and

$R''_1$ represents a hydrogen atom, an alkyl or hydroxyalkyl radical each having from 1 to 5 carbon atoms, an alkenyl radical having from 2 to 5 carbon atoms or a cycloalkyl or cycloalkenyl radical each having from 3 to 7 carbon atoms;

characterized in that the corresponding amide of the general formula VI:

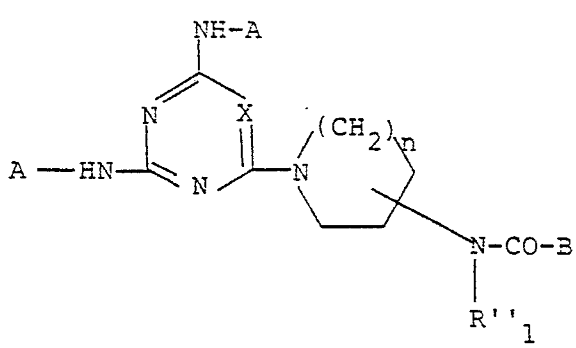

(VI)

in which A, X, n, $R''_1$ and B have the meanings defined above, is reduced by means of a suitable hydride.

4. A process for the preparation of di-substituted polymethyleneimines corresponding to the general formula I''':

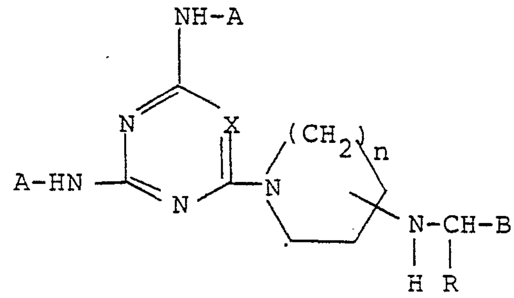

(I''')

in which A, X, n, R and B have the meanings defined in claim 1, characterized in that a mixture of a ketone of the general formula VII :

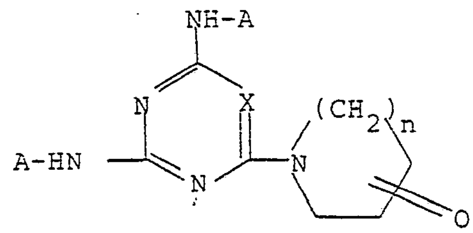

(VII)

in which A, X and n are as defined in claim 1, and an amine of the general formula VIII :

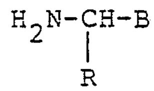

(VIII)

in which R and B have the meanings defined in claim 1, is treated with sodium cyanoborohydride.

**Patentansprüche** (für die Vertragsstaaten : BE, CH, DE, FR, GB, IT, LI, LU, NL, SE)

1. Disubstituierte Polymethylenimine der allgemeinen Formel I

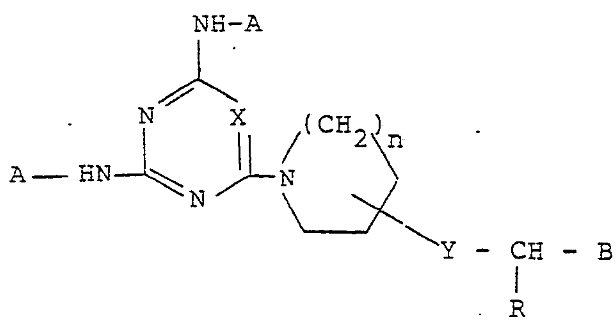

(I)

in der

A eine Propyl-, Allyl- oder Buten-2-yl-gruppe ;

X eine —CH-Gruppe oder ein Stickstoffatom ;

n 0 oder eine ganze Zahl mit einem Wert von 1 oder 2 ;

Y ein Sauerstoffatom oder eine Gruppe der Formel N-$R_1$, in der $R_1$ ein Wasserstoffatom, eine Alkyl- oder Hydroxyalkylgruppe mit jeweils 1 bis 5 Kohlenstoffatomen, eine Alkenylgruppe mit 2 bis 5 Kohlenstoffatomen, eine Cycloalkyl- oder Cycloalkenylgruppe mit jeweils 3 bis 7 Kohlenstoffatomen oder eine Acetylgruppe ;

R ein Wasserstoffatom, eine Alkylgruppe mit 1 bis 5 Kohlenstoffatomen, eine Cycloalkylgruppe mit 5 bis 7 Kohlenstoffatomen oder eine gegebenenfalls durch ein oder mehrere Fluor- oder Chloratome substituierte Phenylgruppe ; und

B ein Wasserstoffatom,

eine Alkylgruppe mit 1 bis 5 Kohlenstoffatomen,

eine Alkenylgruppe mit 2 bis 5 Kohlenstoffatomen,

eine Phenyl-, Naphthyl-, Benzofuranyl-, Benzothienyl-, Benzodioxolyl-, Benzodioxanyl-, Benzodioxi-

nyl-, $\Delta_3$-Chromenyl-, Thiochromenyl-, oder Chromanylgruppe, die gegebenenfalls durch ein oder mehrere Fluor- oder Chloratome, Alkyl- oder Alkoxygruppen mit jeweils 1 bis 5 Kohlenstoffatomen substituiert sein können, oder

eine Gruppe der Formel

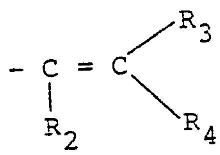

in der $R_2$, $R_3$ und $R_4$, die gleichartig oder verschieden sein können, jeweils Wasserstoffatome oder eine gegebenenfalls durch ein oder mehrere Chlor- oder Fluoratome substituierte Phenylgruppe darstellen, bedeuten.

2. Die Salze der Verbindungen gemäß Anspruch 1 mit geeigneten Säuren.

3. Die Salze nach Anspruch 2, die physiologisch verträglich sind.

4. 1-(4,6-Bisallylamino-s-triazin-2-yl)-4-(bis-p-fluorbenzhydrylamino)-piperidin und dessen Difumarat.

5. 1-(4,6-Bisallylamino-s-triazin-2-yl)-4-p-fluorbenzhydrylamino-piperidin und dessen Dihydrochlorid.

6. 1-(4,6-Bisallylamino-s-triazin-2-yl)-4-ethoxy-piperidin und dessen Difumarat.

7. 1-(4,6-Bisallylamino-s-triazin-2-yl)-4-(benzofuran-2-yl-methylamino)-piperidin.

8. 1-(4,6-Bisallylamino-s-triazin-2-yl)-4-p-fluor-cinnamylamino-piperidin und dessen Difumarat.

9. 1-(2,4-Bisallylamino-pyrimidin-6-yl)-4-p-fluor-cinnamylamino-piperidin und dessen Difumarat.

10. Verfahren zur Herstellung der Verbindungen nach Anspruch 1, dadurch gekennzeichnet, daß man ein disubstituiertes Polymethylenimin der allgemeinen Formel II

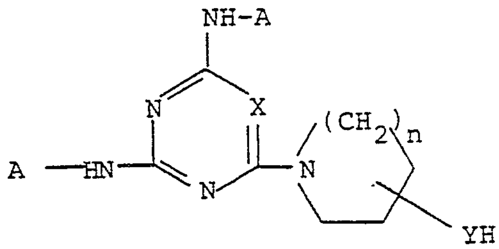

(II)

in der A, n, X und Y die in Anspruch 1 angegebenen Bedeutungen besitzen, mit einem Halogenderivat der allgemeinen Formel III

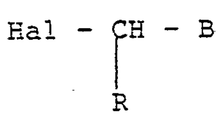

(III)

in der R und B die in Anspruch 1 angegebenen Bedeutungen besitzen und Hal für ein Chlor- oder Bromatom steht, kondensiert.

11. Verfahren zur Herstellung der Verbindungen nach Anspruch 1 der allgemeinen Formel I'

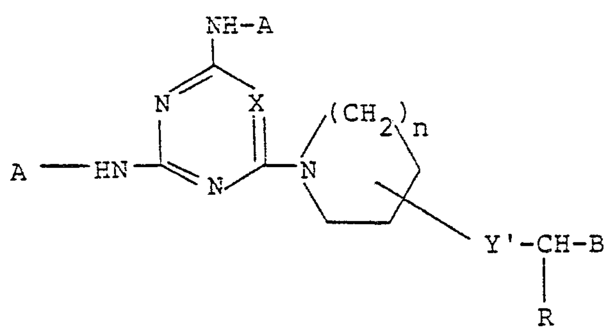

(I')

in der A, n, R und B die in Anspruch 1 angegebenen Bedeutungen besitzen und

Y' ein Sauerstoffatom oder eine Gruppe der Formel N-R'$_1$, worin R'$_1$ für eine Alkyl- oder Hydroxyalkylgruppe mit jeweils 1 bis 5 Kohlenstoffatomen, eine Alkenylgruppe mit 2 bis 5 Kohlenstoffatomen, eine Cycloalkyl- oder Cycloalkenylgruppe mit jeweils 3 bis 7 Kohlenstoffatomen oder eine Acetylgruppe darstellt, bedeutet, dadurch gekennzeichnet, daß man ein Halogenderivat der allgemeinen Formel IV

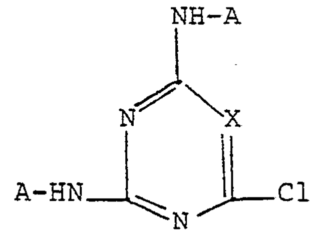

(IV)

in der A und X die oben angegebenen Bedeutungen besitzen, mit einem monosubstituierten Polymethylenimin der allgemeinen Formel V

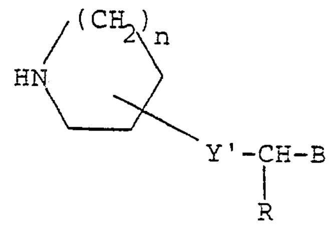

(V)

in der n, R und Y' die oben angegebenen Bedeutungen besitzen, kondensiert.

12. Verfahren zur Herstellung der Verbindungen nach Anspruch 1 der allgemeinen Formel I''

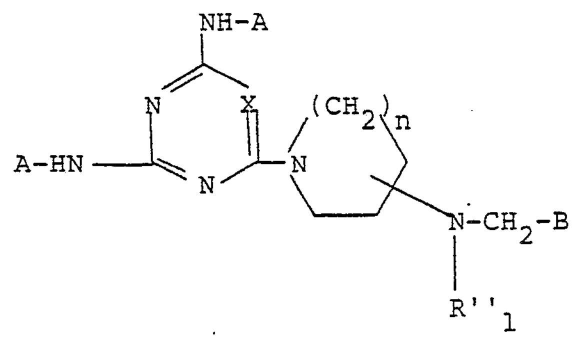

(I'')

in der A, X, n und B die in Anspruch 1 angegebenen Bedeutungen besitzen und

$R''_1$ ein Wasserstoffatom, eine Alkyl- oder Hydroxyalkylgruppe mit 1 bis 5 Kohlenstoffatomen, eine Alkenylgruppe mit 2 bis 5 Kohlenstoffatomen oder eine Cycloalkyl- oder Cycloalkenylgruppe mit jeweils 3 bis 7 Kohlenstoffatomen bedeutet,

dadurch gekennzeichnet, daß man das entsprechende Amid der allgemeinen Formel VI

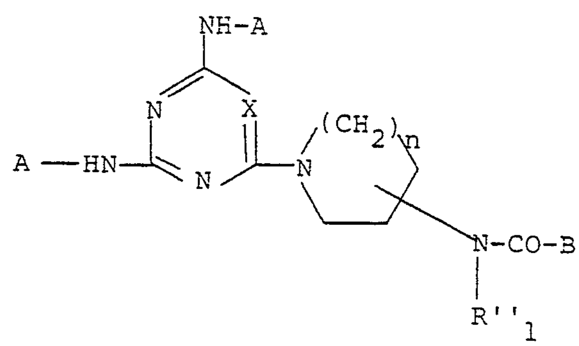

(VI)

in der A, X, n, $R''_1$ und B die oben angegebenen Bedeutungen besitzen, mit einem geeigneten Hydrid reduziert.

13. Verfahren zur Herstellung der Verbindungen nach Anspruch 1 der allgemeinen Formel I'''

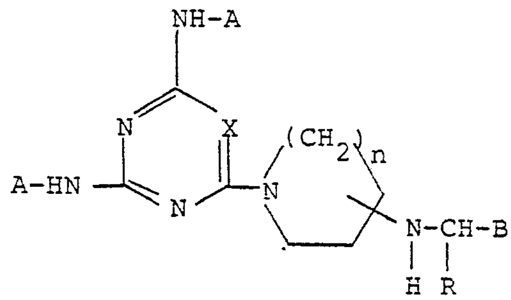

(I''')

in der A, X, n, R und B die in Anspruch 1 angegebenen Bedeutungen besitzen, dadurch gekennzeichnet, daß man eine Mischung aus einem Keton der allgemeinen Formel VII

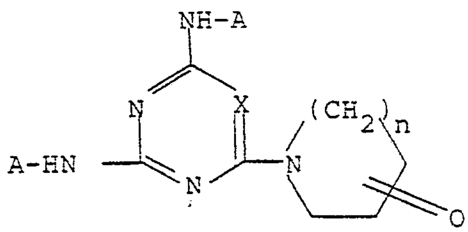

(VII)

in der A, X und n die in Anspruch 1 angegebenen Bedeutungen besitzen, und einem Amin der allgemeinen Formel VIII

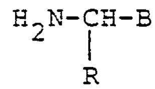

(VIII)

in der R und B die in Anspruch 1 angegebenen Bedeutungen besitzen, mit Natriumcyanoborhydrid behandelt.

14. Pharmazeutische Zubereitungen enthaltend als Wirkstoff eine Verbindung nach einem der Ansprüche 1 oder 3 bis 9 und geeignete pharmazeutische Trägermaterialien.

15. Pharmazeutische Zubereitungen nach Anspruch 14, dadurch gekennzeichnet, daß sie in einer insbesondere zur Behandlung von Gewebehypoxien geeigneten Form vorliegen.

**Patentansprüche** (für den Vertragsstaat AT)

1. Verfahren zur Herstellung von disubstituierten Polymethyleniminen der allgemeinen Formel I

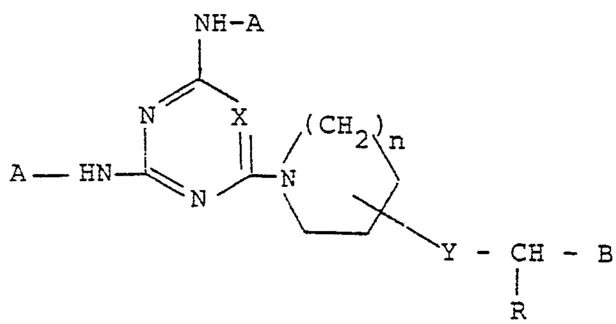

(I)

in der

A eine Propyl-, Allyl- oder Buten-2-yl-gruppe ;

X eine —CH-Gruppe oder ein Stickstoffatom ;

n 0 oder eine ganze Zahl mit einem Wert von 1 oder 2 ;

Y ein Sauerstoffatom oder eine Gruppe der Formel N-$R_1$, in der $R_1$ ein Wasserstoffatom, eine Alkyl- oder Hydroxyalkylgruppe mit jeweils 1 bis 5 Kohlenstoffatomen, eine Alkenylgruppe mit 2 bis 5 Kohlenstoffatomen, eine Cycloalkyl- oder Cycloalkenylgruppe mit jeweils 3 bis 7 Kohlenstoffatomen oder eine Acetylgruppe ;

R ein Wasserstoffatom, eine Alkylgruppe mit 1 bis 5 Kohlenstoffatomen, eine Cycloalkylgruppe mit 5

mit 7 Kohlenstoffatomen oder eine gegebenenfalls durch ein oder mehrere Fluor- oder Chloratome substituierte Phenylgruppe ; und

B ein Wassserstoffatom,

eine Alkylgruppe mit 1 bis 5 Kohlenstoffatomen,

eine Alkenylgruppe mit 2 bis 5 Kohlenstoffatomen,

eine Phenyl-, Naphthyl-, Benzofuranyl-, Benzothienyl-, Benzodioxolyl-, Benzodioxanyl-, Benzodioxinyl-, $\Delta_3$-Chromenyl-, Thiochromenyl- oder Chromanylgruppe, die gegebenenfalls durch ein oder mehrere Fluor- oder Chloratome, Alkyl- oder Alkylgruppen mit jeweils 1 bis 5 Kohlenstoffatomen substituiert sein können, oder

eine Gruppe der Formel

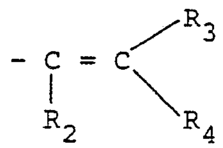

in der $R_2$, $R_3$ und $R_4$, die gleichartig oder verschieden sein können, jeweils Wasserstoffatome oder eine gegebenenfalls durch ein oder mehrere Chlor- oder Fluoratome substituierte Phenylgruppe darstellen, bedeuten,

und ihrer Säureadditionssalze mit geeigneten Säuren, dadurch gekennzeichnet, daß man ein disubstituiertes Polymethylenimin der allgemeinen Formel II

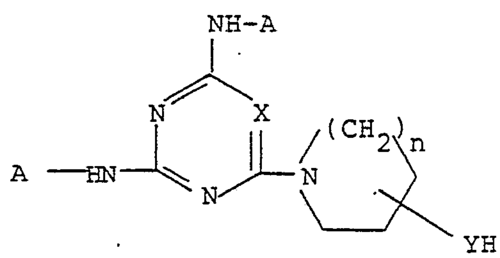

(II)

in der A, n, X und Y die oben angegebenen Bedeutungen besitzen, mit einem Halogenderivat der allgemeinen Formel III

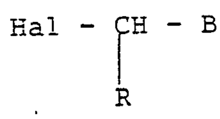

(III)

in der R und B die oben angegebenen Bedeutungen besitzen und Hal für ein Chlor- oder Bromatom steht, kondensiert, und gewünschtenfalls die in dieser Weise erhaltenen Derivate I mit geeigneten Säuren in die entsprechenden Additionssalze überführt.

2. Verfahren zur Herstellung von disubstituierten Polymethyleniminen der allgemeinen Formel I′

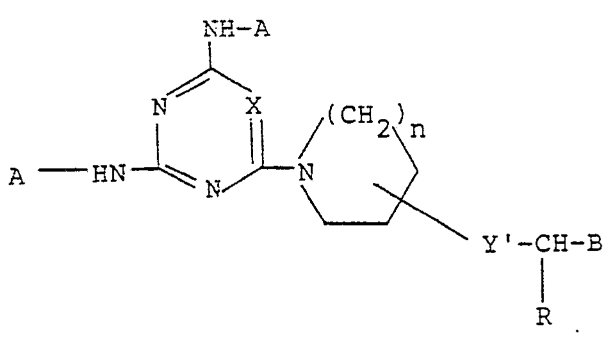

(I′)

in der A, n, R und B die in Anspruch 1 angegebenen Bedeutungen besitzen und

Y′ ein Sauerstoffatom oder eine Gruppe der Formel N-$R'_1$, worin $R'_1$ für eine Alkyl- oder Hydroxyalkylgruppe mit jeweils 1 bis 5 Kohlenstoffatomen, eine Alkenylgruppe mit 2 bis 5 Kohlenstoffatomen, eine Cycloalkyl- oder Cycloalkenylgruppe mit jeweils 3 bis 7 Kohlenstoffatomen oder eine Acetylgruppe darstellt, bedeutet, dadurch gekennzeichnet, daß man ein Halogenderivat der allgemeinen Formel IV

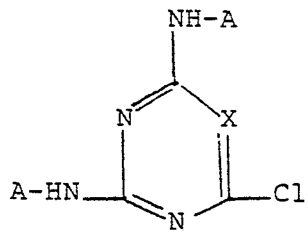

(IV)

in der A und X die in Anspruch 1 angegebenen Bedeutungen besitzen, mit einem monosubstituierten Polymethylenimin der allgemeinen Formel V

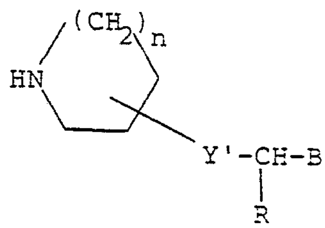

(V)

in der n, R und Y' die oben angegebenen Bedeutungen besitzen, kondensiert.

3. Verfahren zur Herstellung von disubstituierten Polymethyleniminen der allgemeinen Formel I"

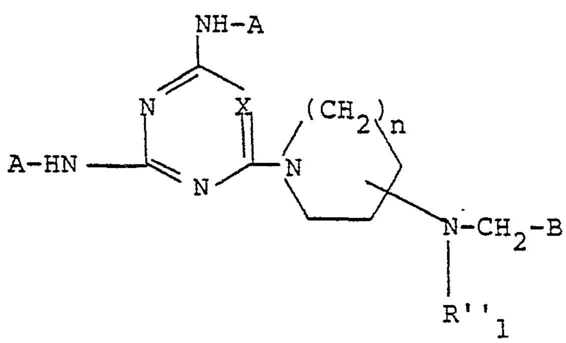

(I")

in der A, X, n und B die in Anspruch 1 angegebenen Bedeutungen besitzen und

$R''_1$ ein Wasserstoffatom, eine Alkyl- oder Hydroxyalkylgruppe mit 1 bis 5 Kohlenstoffatomen, eine Alkenylgruppe mit 2 bis 5 Kohlenstoffatomen oder eine Cycloalkyl- oder Cycloalkenylgruppe mit jeweils 3 bis 7 Kohlenstoffatomen bedeutet,
dadurch gekennzeichnet, daß man das entsprechende Amid der allgemeinen Formel VI

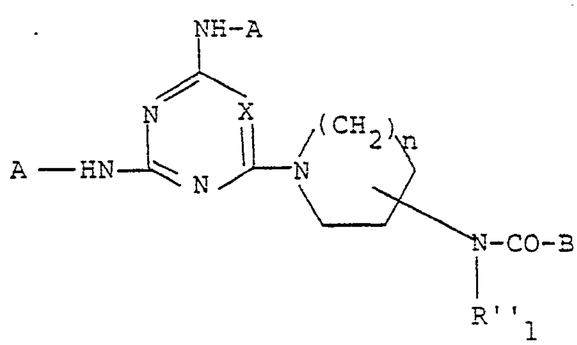

(VI)

in der A, X, n, $R''_1$ und B die oben angegebenen Bedeutungen besitzen, mit einem geeigneten Hydrid reduziert.

4. Verfahren zur Herstellung von disubstituierten Polymethyleniminen der allgemeinen Formel I'"

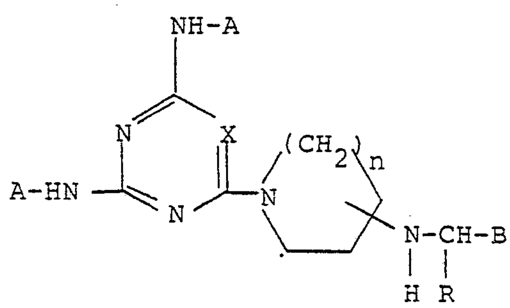

(I''')

in der A, X, n, R und B die in Anspruch 1 angegebenen Bedeutungen besitzen, dadurch gekennzeichnet, daß man eine Mischung aus einem Keton der Allgemeinen Formel VII

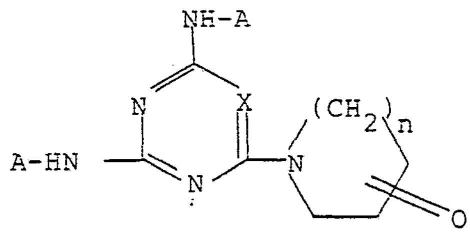

(VII)

in der A, X und n die in Anspruch 1 angegebenen Bedeutungen besitzen, und einem Amin der allgemeinen Formel VIII

$$H_2N-CH(R)-B \qquad (VIII)$$

in der R und B die in Anspruch 1 angegebenen Bedeutungen besitzen, mit Natriumcyanoborhydrid behandelt.